# EUROPEAN PATENT APPLICATION

(11) **EP 2 031 071 A1**
(43) Date of publication of application: **04.03.2009**
(21) Application number: 07017102.0
(22) Date of filing: 31.08.2007
(51) Int. Cl.: C12Q 1/68

(54) **Method for categorizing samples containing spermatozoa by molecular profiling**

(71) Applicant: Minitüb Abfüll- und Labortechnik GmbH & Co. KG, 84184 Tiefenbach (DE)
(72) Inventor: Simmet, Christian, 84036 Landshut (DE); Wolf, Eckhard, 85256 Vierkirchen (DE); Blum, Helmut, 81475 München (DE); Bauersachs, Stefan, 81245 München (DE); Boelhauve, Marc, 85521 Ottobrunn (DE)
(74) Representative: Engelhard, Markus

(57) **Abstract**

The present invention relates to methods for categorizing samples containing spermatozoa by obtaining a RNA profile in said sample by hybridization and/or sequencing techniques, wherein the RNA is preferably selected from messenger RNA (mRNA), noncoding RNA (ncRNA) and micro RNA (miRNA). The present invention relates further to the use of RNA profiles and/or translation product profiles as selection criterions, such as fertility and breeding selection, of the sample donor. The present invention allows for distinguishing male and female spermatozoa of a sample and subsequently separating male and female spermatozoa. With the methods of the invention categorized samples as well as male and female spermatozoa can be obtained.

## Description

The present invention relates to a method for categorizing samples containing spermatozoa by obtaining a RNA profile in said sample by hybridization and/or sequencing techniques, wherein the RNA is preferably selected from messenger RNA (mRNA), noncoding RNA (ncRNA) and micro RNA (miRNA). The present invention relates further to the use of RNA profiles and/or translation product profiles as selection criterions, such as fertility and breeding selection, of the sample donor. The present invention allows for distinguishing male and female spermatozoa of a sample and subsequently separating male and female spermatozoa. With the methods of the invention categorized samples as well as male and female spermatozoa can be obtained.

### Background of the invention

The spermatozoon is a highly differentiated and specialized cell, which until recently was thought only to transport the paternal genome into the oocyte. Several reports in the past years describe the presence of various RNAs in the male gametes of several species (reviewed in Krawetz, 2005; Miller et al., 2005). RNA carriage by mature spermatozoa is an interesting finding, because nuclear gene expression is progressively shutdown during spermiogenesis in the haploid phase of spermatogenesis to allow substitution of histones by the highly charged and physically smaller protamines (PRM1 and PRM2), facilitating further compaction of the haploid genome (Balhorn et al., 1999). To overcome the shutdown of nuclear transcription, developing spermatids rely on translational control of stored mRNAs to produce the proteins necessary for chromatin re-compaction and to finalize the differentiation of the spermatozoon (Steger, 2001; Miller and Ostermeier, 2006). Mature spermatozoa do not contain sufficient 28S or 18S rRNAs to support translation, i.e. essential components necessary to support a translational machinery are absent, indicating their removal or breakdown during spermatozoal maturation and, by prolongation, the probably selective retention of mRNA species (Miller et al., 1999).

Spermatozoa of a normal human ejaculate retain about 5000 mRNA types (Miller and Ostermeier, 2006). A serial analysis of gene expression (SAGE) tag map of human spermatozoal RNA has been reported that identified 2712 and 2459 unique transcripts from pooled and individual ejaculate samples, respectively (Zhao et al., 2006), containing for example GA17 (PCI domain containing 1, also known as dendritic cell protein); COX5B (cytochrome c oxidase subunit Vb), a subunit of the terminal mitochondrial respiratory transport enzyme; TFAM (transcription factor A-mitochondrial), a mitochondrial transcription factor; and small RNA-binding proteins.

In addition to messenger RNAs (mRNAs) encoding protein products, microRNAs (miRNAs) and noncoding RNAs may be important for sperm function. Discovered just over a decade ago, miRNA is now recognized as one of the major regulatory gene families in eukaryotic cells. Hundreds of miRNAs have been found in animals, plants and viruses. Through specific base-pairing with mRNAs, these tiny -22-nt RNAs induce mRNA degradation or translational repression, or both. Because a miRNA can target numerous mRNAs, often in combination with other miRNAs, miRNAs operate highly complex regulatory networks (reviewed in Kim & Nam, 2006). The methods for miRNA discovery have been summarized recently and include forward genetics approaches, the sequencing of small RNA libraries, and the computational prediction of miRNA genes (Berezikov et al., 2006). Further, strategies for the functional analysis of miRNAs (Krützfeldt et al., 2006) and for the prediction of their target genes (Rajewsky, 2006) are being developed. Although clear roles of miRNAs have been identified in many biological processes, e.g. in cancer (Esquela-Kerscher & Slack, 2006), only little information is available regarding potential involvement of miRNAs in spermatogenesis and sperm function.

First evidence that the microRNA pathway may be involved in the control of postmeiotic male germ cell differentiation came from studies in the mouse. The so-called chromatid body, a finely filamentous, lobulated perinuclear granule located in the cytoplasm of mammalian postmeiotic round spermatids, was found to contain Dicer and components of microRNP complexes (including Ago proteins and microRNAs) in a highly concentrated form (Kotaja et al., 2006). Thus, the authors suggested the chromatoid body as an "intracellular nerve center" of the microRNA pathway.

Another class of noncoding small RNAs (piRNAs = PIWI-interacting RNAs) has been shown to interact with MIWI (a murine member of the family of PIWI/Argonaute proteins), a cytoplasmic protein present in spermatocytes and round spermatids, which is required for the expression of its target mRNAs involved in spermiogenesis (Grivna et al., 2006).

A first report of miRNAs and of small noncoding antisense RNAs in mature spermatozoa was described by Ostermeier et al. (2005). In this case, RNA from sperm of 6 normal fertile men was directly hybridized to sense oligonucleotide arrays containing 10 000 elements, thus no relation to fertility was demonstrated.

In the mouse, miRNAs were reported to be present in sperm structures that enter the oocyte at fertilization (Amanai et al. 2006). The sperm contained a broad profile of miRNAs and a subset of potential mRNA targets, which were expressed in fertilizable metaphase II (mII) oocytes. The levels of sperm-borne miRNA (measured by quantitative PCR) were low relative to those of unfertilized mII oocytes, and fertilization did not alter the mII oocyte miRNA repertoire that included the most abundant sperm-borne miRNAs. The authors concluded that sperm-borne prototypical miRNAs play a limited role, if any, in mammalian fertilization or early preimplantation development.

### Infertility research

Hypothesizing that spermatozoal RNA offers a snapshot of the spermatogenic potential of the testis, the resource can be used for infertility research (Miller and Ostermeier, 2006). However, an infertile phenotype affecting the semen profile is rarely obvious, given the natural heterogeneity of human semen profiles and single gene defects that may cause it are unlikely to have any clear effect unless the genotype is homozygous (Miller and Ostermeier, 2006).

But predicting the fertility or infertility of a male is very useful in a variety of contexts. For example, the artificial insemination industry is interested in knowing the likelihood that fertilization will occur if a female is artificially inseminated with a particular male's semen.

Some reports have already appeared aimed at testing the utility of spermatozoal RNA as a molecular resource for infertility investigation. Spermatozoal motility, e.g. which is usually an indicator of male fertility, appears to carry a molecular signature that can be detected using simple RT-PCR-based tests. A study using microarray-based data coupled with real-time PCR detected quantitative changes in the presence of several spermatozoal mRNAs relating to the motility of the sampled population (Wang et al., 2004). These reports highlight two important findings. First, spermatozoal RNA appears to reflect the inter-sample non-affected versus affected phenotype (at least for motility). Second, intra-sample variations in transcript presence associate with spermatozoal morphology on the basis of buoyant density.

US 2003/0108925 A1 discloses genetic testing for male infertility or damage to spermatozoa by providing a microarray of DNA probes with a sample of spermatozoa to determine the mRNA fingerprints of the sample and comparing the mRNA fingerprints of the sample with the mRNA fingerprints of normal fertile male spermatozoa. Messenger RNAs were isolated from testes and ejaculate spermatozoa, and the corresponding cDNAs were hybridized to a series of microarrays containing 30,892 Expressed Sequence Tag probes (ESTs), of which 27,016 are unique. Human Genefilter® microarrays 200, 201, 202, 203, 204 and 211 (Research Genetics) were used, which, however, cover the entire human genome.

US 2006/0199204 A1 (a continuation-in part application of US 2003/0108925 A1) discloses a method for distinguishing between sperm from normal and affected individuals comprising: (a) providing a list of transcripts found in normal fertile sperm that are consistently detected across array platforms; (b) obtaining a list of transcripts from sperm of a subject and compiling a list of high confidence transcripts; (c) comparing the list of high confidence transcripts from sperm of a subject with the list of transcripts found in normal sperm; and (d) if the list of high confidence transcripts from the sperm of the subject is similar to that of the list of transcripts found in normal sperm, the subject is normal; if not, the subject is affected. Array platforms used were the Affymetrix Human U133 plus 2 and Illumina Centrix Human Expression BeadChip platforms. The results obtained for both platforms for the normal fertile individuals were then compared to the results obtained in US 2003/0108925 A1 based upon the Research Genetics spotted cDNA microarray platform.

### Gender preselection

The ability to specify male or female offspring is very useful in a variety of contexts, especially in livestock industry. Planning the sex of cattle offspring is already practiced on a limited basis. This procedure consists of removing embryos from the cow, identifying their potential gender and re-implanting only those of the desired sex (post-fertilization analysis and selection). Sexing of preimplantation embryos has been accomplished by (a) karyotyping, (b) by polymerase chain reaction (PCR) amplification of Y-chromosome specific nucleotide sequences, or (c) by immunological methods. The first two methods suffer from the disadvantage that they involve embryo micromanipulation in order to obtain biopsies and therefore they are "invasive" procedures. The only method used routinely on a commercial scale is to biopsy embryos and amplify Y-chromosome-specific DNA using the polymerase chain reaction (Aasen et al., 1990). This method is effective for more than 90% of embryos and is > 95% accurate.

Within males, X- and Y-bearing spermatozoa are essentially identical phenotypically due to: (1) connection of spermatogenic cells by intercellular bridges, (2) transcriptional inactivation of sex chromosomes during meiosis and spermiogenesis, (3) severe limitation of all gene expression during the later stages of spermiogenesis, and (4) coating of all spermatozoa with common macromolecules during and after spermiogenesis (Seidel et al., 1999). One consequence is that no convincing phenotypic difference has been detected between X- and Y-chromosome-bearing spermatozoa.

Many techniques have been investigated for the separation of X- from Y-chromosome-bearing sperm in mammals. Some techniques have been based on the characteristics of the sperm e.g. size, head shape, mass, surface properties, surface macromolecules, DNA content, swimming velocity, and motility (see overview in Gledhill 1988; Seidel et al., 1999). The only established and measurable difference between X and Y sperm that is known and has been proved to be scientifically valid is their difference in deoxyribonucleic acid (DNA) content (Johnson et al., 1999). The X chromosome is larger and contains slightly more DNA than the Y chromosome. The difference in total DNA between X-bearing sperm and Y-bearing sperm is 3.4% in boar, 3.8% in bull, and 4.2% in ram sperm. Up to now the process of flowcytometric sorting of spermatozoa according to this criterion is very slow (about 20 million sperms separated per hour), the recovery rate is very poor as the majority of the sorted spermatozoa cannot be categorized correctly and have to be wasted, not all bulls/boars are suitable to this limited process. Furthermore the numbers of piglets (originating from inseminations with flow-cytometric sorted sperm) were reduced (Rath et al., 1999).

Furthermore, WO 01/32008 A1 discloses methods for the control of sex ratio in non-human mammals. These methods involve the production of transgenic animals which have particular transgenes integrated into their genomes, wherein at least one transgene selectively inhibits the function of those sperm having a specified sex chromosome type. Animals produced using such methods are also provided, as are the transgene constructs.

WO 01/47353 A1 discloses a method for controlling the sex ratio of offspring by targeting transgenes. The method involves: (a) selecting or creating a transgene whose expression can interfere with sperms ability to undergo fertilization, and whose gene products (mRNA and protein) do not diffuse freely among inter-connected spermatids; (b) placing the transgene under the regulatory control of post-meiotic spermatogenesis-specific promoter; and (c) using the transgene to generate transgenic animals in the way that the transgene is inserted onto one of the two sex chromosomes.

WO 02/077637 A1 discloses methods and materials for pre-selecting the sex of mammalian offspring. In particular, the materials and methods described herein permit the enrichment of X- or Y-chromosome-bearing sperm in semen by introducing a transgene into a sex chromosome under control of regulatory sequences that provide for expression of the transgene in a haploid-specific manner.

Thus, there is a need in the art for methods and tools that allow identifying of semen donors, such as breeding animals, or of semen samples based on respective desirable characteristics, such as fertility, which subsequently facilitates directed selection in animal breeding and other areas of livestock industry. There is furthermore a need in the art for methods and tools that allow sexing of semen samples.

Therefore, the problem to be solved by this invention was to improve the methods and tools known in the art of breeding selection and provide methods which allows the fast and simple identification of semen samples and which allows the categorization of these samples.

The problem is solved by the present invention by providing a method for categorizing a sample containing spermatozoa.

The method for categorizing a sample containing spermatozoa according to the present invention comprises determining a RNA expression profile in said sample by hybridization and/or sequencing techniques.

A "RNA profile" or "RNA expression profile" (both terms may be used alternately throughout the description) is the expression profile of all detectable RNA species or a subset thereof and is preferably selected from the expression profile of messenger RNA (mRNA), noncoding RNA (ncRNA) and micro RNA (miRNA).

With respect to mRNA, determining a RNA expression profile of full length mRNAs as well as partial sequences thereof is comprised.

Furthermore, the RNA expression profiles of other noncoding RNA, preferably small noncoding RNA, such as piRNA (PIWI-interacting RNA) is also comprised within the scope of the present invention.

Determining a RNA expression profile according to the present invention comprises determining one or more of the following:
- presence, frequency and/or concentration of RNA,
- differences in sequence, such as deletions, polymorphisms and SNP genotyping,
- differences in RNA length,
- alternative usage of exons and introns.

The hybridization is preferably carried out by using nucleic acid arrays, preferably cDNA arrays or oligonucleotide arrays.

The sequencing techniques are preferably selected from sequencing of normalized cDNA libraries and sequencing of the whole profile by high-throughput sequencing technologies, such as 454 technology.

Determining a RNA expression profile furthermore preferably comprises a quantitative analysis, such as quantitative analysis of candidate RNAs, such as by determining the relative abundance of the candidate RNAs. The relative decrease or increase of RNA concentrations is preferably determined by comparison to a sample of a donor with known characteristics, such as desired fertility. Preferred means are quantitative PCR methods.

Thus, the methods of the invention comprise the assessment of the expression profile of messenger RNAs (mRNA, full length, partial sequences), non-coding RNAs (ncRNAs) or micro RNAs (miRNA) by hybridization of cDNA arrays or oligonucleotide arrays, or by sequencing techniques.

The following RNA species/parameters are preferably included in the assessment: concentration of messenger RNA (mRNA), noncoding RNA (ncRNA), microRNA (miRNA); polymorphisms on the level of mRNA (e.g. single nucleotide polymorphisms = SNPs).

Preferred determinations on the level of mRNA are:
- presence, frequency and/or concentration of the mRNA,
- alternative usage of exons and introns (splice variants),
- differences in mRNA length,
- differences in nucleotide sequence (e.g., SNPs).

Differences in mRNA length which can be determined are, for example, caused by different 5' and/or 3' regions.

Preferred determinations on the level of miRNA are:
- presence, frequency and/or concentration of miRNA,
- differences in miRNA sequence (e.g., SNPs)

Preferred determinations on the level of ncRNA are:
- presence, frequency and/or concentration of ncRNA,
- differences in ncRNA sequence (e.g., SNPs)

Preferred methods for the assessment/determination of RNA expression profiles according to the present invention are:
Hybridisation (cDNA arrays or oligonucleotide arrays),
Sequencing of normalized cDNA libraries,
Sequencing of the whole profile by high-throughput sequencing technologies (e.g., 454 technology),
Quantitative analysis of candidate RNAs.

In a preferred embodiment the method of categorizing a sample containing spermatozoa according to the present invention combines hybridization techniques, such as arrays, with sequencing techniques, such as SNPs, exon/intron usage.

### Samples and donors

A "sample containing spermatozoa" according to the invention is an ejaculate, semen, a sperm-rich fraction of ejaculate, sperm cells from the epidydimis, sperm cells or their progenitors from the testis.

Furthermore, processed and packaged semen destined for artificial insemination (AI) or *in vitro* fertilization (IVF) as well as sperms derived by the swim-up procedure are also samples containing spermatozoa according to the invention.

In addition, samples containing spermatozoa according to the invention can also be the above sperm samples that are pre-treated and/or processed. Examples for such pre-treatment and/or processing are dilution, concentration, centrifugation, purification, labelling, drying, bottling, freezing, storing.

Thus, samples containing spermatozoa according to the invention can be sperms that are processed by a Percoll gradient centrifugation or density gradient centrifugation, as well as sperm samples that are bottled and/or stored, such as in semen straws, tubes, bottles and other suitable containers.

Samples containing spermatozoa according to the invention are not intended to be limited with respect to state (liquid/frozen) or age (fresh/stored). Thus, liquid samples, frozen samples, fresh samples, stored samples etc. are all within the meaning of samples containing spermatozoa according to the invention.

Preferably, the donor of the sample containing spermatozoa is an animal, a mammal, a bird, livestock or a breeding animal.

The donor of the sample containing spermatozoa is preferably a boar, a bull, a stallion, a rooster, a male dog, a male.

### Embodiments of the method of the present invention

In a first step a) of a preferred embodiment of the method according to the present invention a sample containing spermatozoa is provided.

In a second step b) of a preferred embodiment of the method according to the present invention the total RNA is isolated from the sample containing spermatozoa.

The isolation of total RNA from the sample preferably comprises one or several of the steps of treating the sample with reagent, such as TRIZOL®, homogenization, chloroform treatment, isopropanol precipitation, centrifugation, DNaseI digestion. It lies within the knowledge of a person of skill in the art to determine the conditions required for a particular sample.

In another embodiment the sample containing spermatozoa is directly utilized and, thus, no RNA isolation step (step b) is carried out. For example, a sample containing spermatozoa, such as a sample with a certain amount of sperms, can be directly subjected to a RT PCR (see step c) below) and subsequently to an amplification, such as a PCR.

In a third step c) of the preferred embodiment of the method according to the present invention cDNA is synthesized from the isolated total RNA.

Preferably, double stranded (ds) cDNA is synthesized. The synthesis of cDNA is preferably carried out by RT PCR. In a preferred embodiment first strand synthesis is carried out by RT PCR and second strand synthesis is carried out using a DNA polymerase and optionally a DNA ligase. Furthermore, suitable primers, reaction mixture, enzymes with reverse transcription activity are used. Suitable components, enzymes and kits are also commercially available and known to a skilled artisan.

Preferred enzymes are Superscript^{™} III reverse transcriptase, *E*. *coli* DNA Polymerase I, *E*. *coli* DNA ligase.

In a subsequent step d) of a preferred embodiment of the method according to the invention labelled probes from the cDNA obtained in step c) are generated.

It is preferred that these probes (also called hybridization probes) are cDNA or cRNA, more preferably single stranded cDNA (ss cDNA), double stranded cDNA (ds cDNA) or single stranded cRNA (ss cRNA). However, also double stranded cRNA (ds cRNA) can be used.

The labelled probes are preferably generated by one or more of the following:
- labelling with radioactive or non-radioactive labels,
- incorporating of dNTPs with reactive side groups, and/or
- incorporating of characteristic nucleotide sequence(s).

Preferably the radioactive label is a radio-isotope which is preferably selected from ³³P or ³²P.

Preferably the non-radioactive label is as fluorescent dye or a luminescent dye. A suitable fluorescent dye is preferably selected from CyDye (such as Cy3 and/or Cy5), AlexaDye.

Another preferred non-radioactive label is selected from biotin, digoxigenin and avidin.

Further suitable radioactive and non-radioactive labels can be determined by a skilled artisan by applying the teachings of the present invention.

The hybridization probes are preferably:
- ss cDNA with a radioactive label, a fluorescent dye label or incorporated dNTPs with reactive side groups, such as aminoallyl dNTPs,
- ds cDNA with radioactive or non-radioactive label(s),
- cRNA with radioactive or non-radioactive label(s), incorporated characteristic nucleotide sequence(s) by utilizing specifically modified primers
- RNA with radioactive or non-radioactive label(s), incorporated by treatment with a reactive dye.

Suitable dNTPs with reactive side groups are e.g. aminoallyl dNTPs or thio dNTPs. Further dNTPs with reactive side groups are known in the art.

Suitable characteristic nucleotide sequence(s) are known to a skilled artisan.

The generation of probes labelled with radioactive or non-radioactive label(s) is preferably carried out by enzymatic or non-enzymatic labelling.

The generation of the radio-isotope labelled probes is preferably carried out by random primed labelling.

In a subsequent step e) of a preferred embodiment of the method according to the invention a nucleic acid array is provided. Nucleic acid arrays, preferably cDNA arrays and oligonucleotide arrays, are known in the art, e.g. Affymetrix-based chips and arrays.

In a preferred embodiment the nucleic acid array is a cDNA array that was obtained from a normalized spermatozoa cDNA library.

A "cDNA array" according to the present invention is a microarray (also commonly known as gene or genome chip, DNA chip, or gene array) with a collection of microscopic cDNA spots, arrayed on a solid surface by covalent attachment to chemically suitable matrices, preferred matrices are nylon membranes or glass.

A "normalized spermatozoa cDNA library" according to the present invention is a library with nearly equimolar quantities of their cDNA species.

The term "normalization" according to the present invention means to equalise the frequencies of high, medium and low abundant nucleic acid species.

The cDNA array obtained from a normalized spermatozoa cDNA library is preferably made from another sample containing spermatozoa which was obtained from the same animal species as the sample provided in step a) of the method.

For example, a cDNA array was made from a normalized spermatozoa cDNA library which was obtained from a whole ejaculate or sperm-rich fraction sample of a boar. This cDNA array is used in the method according to the present invention to categorize other samples from boars, such as ejaculates.

The cDNA array obtained from a normalized spermatozoa cDNA library is preferably generated for each animal species to be tested.

The preferred steps necessary for obtaining a normalized spermatozoa cDNA library and subsequently a cDNA array are discussed below.

The cDNA array is preferably made by
- providing a sample containing spermatozoa,
- isolating total RNA from said sample,
- synthesizing cDNA from the RNA isolated,
- normalization of the cDNA obtained,
- cloning of the normalized cDNA into a plasmid vector,
- propagation of individual clones,
- determining the nucleotide sequence of cloned cDNA fragments,
- selection of a representative clone collection by discarding multiple occurring clones,
- preparation of cloned cDNA fragments,
- spotting of cDNAs onto a carrier material or matrix,
- denaturing double stranded cDNA (ds cDNA) and fixation of single stranded cDNA (ss cDNA) on the carrier material or matrix.

The carrier material or matrix is preferably a solid surface, such as a nylon membrane, glass or plastic. Further suitable carrier materials and or matrices are known in the art.

In a more preferred embodiment of the method of the present invention the following features are combined (exemplary advantages stated in parentheses):
1. Utilizing a cDNA array (all RNA species present in a tissue can be analyzed, their sequences do not have to be known).
2. The cDNA array was obtained from a normalized spermatozoa library (also RNA species which are less abundant can be detected more easily; specific for spermatozoa tissue).
3. The carrier material or matrix of the cDNA array is nylon membrane (higher binding capacity of the nylon membrane compared to glass; results e.g. in increased detection range of highly expressed RNA species).
4. Radioactively labelled probed are used (Increase of detection sensitivity; results e.g. in increased detection range of lowly expressed RNA species).

In a preferred embodiment of the method of the present invention, as exemplified above: Due to the utilization of a normalized cDNA library, which is furthermore a specific spermatozoa cDNA library, the method allows a more sensitive and holistic detection of differentially expressed RNA species (mRNA, ncRNA etc) than commercially available arrays (e.g. Affymetrix GeneChips). Due to the use of radioactively labelled probes, rare transcripts can be more easily detected. This sensitive approach allows further the detection of RNA species which are not detectable by commercially available arrays (e.g. Affymetrix GeneChips). The difference in their relative abundance can furthermore be quantified due to the high dynamic range of the method and the direct correlation between bound labelled probe and the concentration of the RNA species in the analyzed tissue.

In a subsequent step f) of a preferred embodiment of the method according to the invention the probes are hybridized with the cDNA array.

Hybridization techniques are well known in the art.

It is, however, preferred to perform the hybridization step in parallel or, if possible, in the same reaction vial in case that several samples are to be categorized by the method according to the invention.

In a subsequent step g) of a preferred embodiment of the method according to the invention a RNA expression profile of the sample is obtained from the hybridization signals.

In a subsequent step h) of a preferred embodiment of the method according to the invention the RNA expression profile of the sample is compared with the RNA expression profile of a control sample.

A "control sample" according to the present invention is derived from an animal of the same species as the donor of the sample to be tested and categorized. Furthermore, the control sample is derived from a healthy animal and an animal with a defined fertility status.

Preferably, the determination of a single RNA expression profile of a (single) control sample is sufficient as reference for comparing gene expression analysis, such as comparison of RNA expression profiles.

The control sample can also be a collection of samples that are characteristic for positive or negative sperm/fertility traits combined with the respective abundance values or occurrence values of an animal with defined fertility status.

In a subsequent step i) of a preferred embodiment of the method according to the invention the sample is categorized according to the RNA expression profile obtained in step g) and/or from the results of the comparison of step h).

Preferably, the sample is categorized with respect to the following parameters: fertility, subfertility, infertility, fecundity, breeding selection, polyspermy and subpopulations of the sample donor.

The term "fertility" according to the present invention is the ability to conceive and have offspring (children). The term "fertility" according to the present invention is furthermore the ability to become pregnant (female) or to determine a pregnancy (male) through normal sexual activity or artificial insemination. The term "fertility" according to the present invention is furthermore the successful production of offspring. Therefore, the development in the potential parents of mature oocytes and sperms are required, followed by sexual intercourse, the opportune encounter between sperm and oocyte in the female's body, fertilization, implantation of the embryo in the uterus, successful prenatal development, and a safe birth. The fertilization process can also be conducted in a laboratory, so called *in vitro* fertilization (IVF), and an *in vitro* culture (IVC) of the fertilized oocyte, possibly until the blastocyst stage. Finally, the cultured embryos are transferred in synchronized recipients. Fertilization is the process of combining sperm and egg to create an embryo.

The term "subfertility" according to the present invention is any form of reduced fertility with prolonged time of unwanted non-conception.
For example, subfertility with respect to humans begins after a term of 12 months (according to WHO) or 24 months (according to ESHRE), respectively, without an established pregnancy.

The term "infertility" according to the present invention refers to the biological inability of a male or a female to contribute to conception. There are many biological causes of infertility, some which may be bypassed with medical intervention.

The term "fecundity" according to the present invention is the physical ability of a male or a female to reproduce. Fecundity is the potential reproductive capacity of an organism or a population.

The term "breeding selection" according to the present invention is the use of controlled reproduction to improve certain characteristics in animals (through the selection of individuals expressing the desired traits). Sperm RNA profiles may serve as novel molecular traits.

The term "polyspermy" according to the present invention is an egg that has been fertilized by more than one sperm (spermatozoon). The term "polyspermy" according to the present invention is furthermore an abnormal condition where the oocyte is fertilized by more than one sperm. Another name for it is polyspermic fertilization, i.e. more than one sperm entering and fertilizing an egg.

The potential clinical utility of spermatozoal RNA is furthermore worth examining because testis biopsy as a route to infertility investigation is generally only undertaken as a last resort. If spermatozoa can provide equivalent information on what underlies a subfertile or infertile phenotype, then a non-invasively obtained semen sample is surely a preferable and more widely acceptable option.

It is furthermore preferred to categorize subpopulations of the sample.

A "subpopulation" according to the present invention is a nearly completely isolated population in the global sperm population. A "subpopulation" according to the present invention is furthermore as completely isolated as required for a further use thereof. A "subpopulation" according to the present invention is furthermore a part or a subdivision or a subset of a sperm population.

Most preferred, a categorization in subpopulations is the type of sex chromosome of the spermatozoa, i.e. X or Y chromosome. Thus, the subpopulations of the sample differ in the type of sex chromosome of the spermatozoa.

Further preferred subpopulations are characterized by the swimming velocity/behaviour (e.g. different sperm populations after a swim-up procedure), different antigens/structures on the sperm surface (sex chromosome specific antigens), different net negative charge of the spermatozoal head.

Thus, in a subsequent optional step j) of the method according to the invention subpopulations of the sample are obtained.

Preferably, the method according to the invention comprises the following steps:
a) providing a sample containing spermatozoa,
b) isolating total RNA from said sample,
c) synthesizing cDNA from the RNA isolated in step b),
d) generating labelled probes from the cDNA obtained in step c),
e) providing a nucleic acid array,
f) hybridizing the probes generated in step d) with the array of step e),
g) obtaining a RNA expression profile of the sample from the hybridization signals obtained in step f),
h) comparing the RNA expression profile of the sample with the RNA expression profile of a control sample,
i) categorizing the sample according to the RNA expression profile obtained in step g) and/or from the results of the comparison of step h),
j) optionally, obtaining subpopulations of the sample.

### Translation product profiles of the sample

It is furthermore preferred that the method according to the present invention comprises the following further steps of;
obtaining a translation product profile of the sample and
categorizing the sample according to the translation product profile.

Preferably, these steps are performed after step g), h) and/or i), i.e. before and/or after categorizing the sample according to the RNA profile.

A "translation product profile" according to the present invention is the qualitative and quantitative protein profile of spermatozoa at a defined stage of spermatogenesis.

The sample is categorized with respect to the parameters discussed above.

### Distinguishing and separating male and female spermatozoa

In a preferred embodiment the method according to the present invention comprises the further step, wherein the male (Y-bearing or Y-chromosome bearing) and female (X-bearing or X-chromosome bearing) spermatozoa of the sample are distinguished from each other.

Preferably, the RNA expression profile and/ore the translation product profile of the sample or aliquots of the sample are used for distinguishing male from female spermatozoa.

In a preferred embodiment the method according to the present invention further comprises step of separating male (Y-bearing or Y-chromosome bearing) from female (X-bearing or X-chromosome bearing) spermatozoa. Preferably, two subpopulations or several aliquots of the sample will be obtained.

The separation of X-and Y-bearing spermatozoa is especially useful for pre-fertilization selection allowing a manipulation of the gender of the offspring.

A further aspect of the present invention is a male (Y-bearing or Y-chromosome bearing) spermatozoon or spermatozoa obtained by a method according to the present invention.

A further aspect of the present invention is a female (X-bearing or X-chromosome bearing) spermatozoon or spermatozoa obtained by a method according to the present invention.

### Categorized samples, subpopulations and spermatozoa

A further aspect of the present invention is a categorized sample obtained by a method according to the present invention.

The category of such a sample is preferably fertility, subfertility, infertility, fecundity, breeding selection, polyspermy, type of sex chromosome, sex of the offspring of the sample donor, as discussed above.

A further aspect of the present invention is a categorized subpopulation of a sample obtained by a method according to the present invention.

Preferred categorized subpopulations are subpopulations of type of sex chromosomes, i.e. X-chromosome bearing and Y-chromosome bearing spermatozoa.

A further aspect of the present invention is a categorized spermatozoon or categorized spermatozoa obtained by a method according to the present invention.

### Uses of RNA profiles and translation product profiles

The above problem is furthermore solved by the present invention by providing the use of a RNA expression profile of a sample containing spermatozoa as selection criterion for fertility, subfertility, infertility, fecundity, breeding selection, and breeding selection based on fertility or for determining the sex chromosome of spermatozoa.

A further aspect of the present invention is the use of a RNA expression profile of a sample containing spermatozoa for obtaining a translation product profile of the sample.

The RNA expression profile is preferably obtained by the methods according to the present invention.

A further aspect of the present invention is the use of a translation product profile of a sample containing spermatozoa as selection criterion for fertility, subfertility, infertility, fecundity, breeding selection, and breeding selection based on fertility or for determining the sex chromosome of spermatozoa.

The translation product profile is preferably obtained by using the RNA expression profile or by the methods according to the present invention.

### RNA profiling of sperm cells

RNA profiling of sperm cells, i.e. obtaining a RNA profile of a sample containing spermatozoa, is a useful tool for the identification and positive selection of males of any species, of semen samples of one or several individuals of a given species or of subpopulations of sperm cells of a given sample. Hence, RNA profiling allows to improve the usage of males, ejaculates or semen cells carrying desired traits.

The same is true for proteins which are encoded by said RNAs, i.e. obtaining a translation product profile of a sample containing spermatozoa.

RNA profiling of sperm cells can also be used for the optimization and further development of technologies used and applied in reproductive biotechnologies in animal breeding. Today, reproductive technologies applied to the semen of animal species rely on the successful use of liquid semen preservation and storage at temperatures between 4°C and 20°C for a storage time of up to 2 weeks, the cryo preservation of semen after freezing or vitrifying a semen sample.

All the above mentioned techniques have produced live and healthy offspring in one or several animal species. However, in many cases the animal breeding industry or scientific community is interested in developing successfully applied techniques further, or in establishing procedures which allow the successful application of preservation techniques not yet usable in today's animal production industry or current gamete preservation scheme for lab animals or wildlife.

The optimization of reproductive technologies in today's animal production industry aims primarily at increasing the efficiency of artificial insemination (A.I.) through better semen preservation, to increase flexibility of semen handling, to increase the fertility of preserved semen or to optimize semen usage in other reproductive technologies like *in vitro* fertilization (IVF).

Bull semen is primarily processed as frozen semen with typically 8 to 20 million sperm cells per insemination dose. Between 20 and 50% of the sperm cells used in one insemination dose do not survive the freeze-thaw procedure and are therefore not able to fertilize the oocyte. Many of the sperm cells which survive the freeze-thaw procedure suffer severe damages in cell compartments essential for fertilization and are therefore also not able to fertilize an oocyte. Thus, it is necessary to identify specific bulls or ejaculates which are more robust and which survive the cryo preservation process in a better way.

Bull semen is also used as liquid semen. In this case the number of sperm cells per dose is lowered to as little as 1 million sperm cells per insemination dose. Good fertilization rates are only achieved when storage time is less than 3 days. Thus, it is necessary to be able to identify specific bulls or ejaculates which are more robust and which survive the storage at ambient temperature in a better way.

Boar semen is primarily used as liquid semen, with the insemination doses stored at +17°C for up to 10 days, maintaining good fertility for that period. However, between 2 to 5 billion sperm cells have to be used per insemination dose to achieve acceptable to good fertility levels in the field. It is known that there is a big variability in boars regarding their fertilizing abilities when highly diluted insemination doses (below 750 million sperm cells/dose) are used. Thus, it is necessary to be able to identify specific boars or ejaculates which are more robust and which survive the storage at a wider temperature range ( i.e. from +5°C to 25°C) for a longer period of time and /or being fertile at high dilution ratios. This will bring more flexibility into the transport chain in terms of requirements for temperature control, it will make transport more cost efficient, it will reduce the frequency of semen production in the A.I. centers and it will provide the possibility of a more intense use of boars with high genetic merits.

Boar semen is also used to a very limited extent as frozen-thawed semen. However, at least twice as many sperm cells are required to produce a pregnancy with frozen-thawed semen when compared to liquid semen. There is also a big variability between boars with regards to their "freezability" It is also known that cryo preserved boar semen has a very short life span inside the female genital tract after AI. RNA profiling can be used to improve cryo preservation techniques and media, to improve media for liquid semen preservation and to select boars with better fertility. The more efficient production of frozen-thawed boar semen with improved fertility will make international business easier as quarantine requirements of the importing countries can easily be met with frozen semen but not with liquid semen. Frozen semen can be tested thoroughly for the absence of pathogens in order to fulfil biosecurity requirements of closed herds and it can also be used as part of a contingency plan in case the supply of liquid semen breaks down.

The same analysis applies to stallion semen.

Rooster semen almost completely looses its fertility when cryo preserved with glycerol, the most widely used cryo protectant. Other cryo protectants like DMSO are not much better. The industry is very interested in using frozen-thawed rooster semen in order to be more flexible with their internationally implemented A.I. programmes or to establish gene banks for their genetic lines.

Advanced breeding companies apply reproductive technologies like IVF where small amounts of semen are used to fertilize oocytes *in vitro*. Polyspermy is a problem in this procedure as the resulting embryos will not develop beyond the blastocyst stage. There is a high variability between males with regards to their ability to produce monospermic embryos in IVF procedures, as well as there is an important interaction between different IVF procedures and males. RNA profiling will be a important tool to identify the most suitable males for this technique.

RNA profiling is also a useful tool for validating semen processing procedures, preservation media or components of media as well as a new materials used in the making of semen containers, like straws or semen tubes, for their suitability to maintain the viability, morphologic and functional integrity as well as fertility of a given semen sample.

### Gender preselection

The ability to specify male or female offspring is very useful in a variety of contexts.

For example, a dairy farmer has little use for most bull calves, the use of sexed semen to produce only females will make milk production more efficient. Swine farmers will be able to produce pork more efficiently if they are able to produce and market only female swine, because males must be caponized several days after birth (Gledhill 1988; Hohenboken 1999; Seidel et al., 1999). In human medicine, the motivation for controlling sexing has been provided by the desire to reduce the incidence of sex-linked genetic disorders (Seidel et al., 1999)

In addition, the ability to specify male or female offspring will shorten the time required for genetic improvements, since desirable traits are often associated with one or the other parent. However, an ability to separate sperm into male- and female- determining groups before they are used for artificial insemination will enhance the overall value of offspring produced by embryo transfer (Seidel et al., 1999). The second approach is the separation of X-and Y-bearing spermatozoa (pre-fertilization selection; Johnson et al., 1999).

The following drawings and examples illustrate the present invention without, however, limiting the same thereto.

### Brief description of the drawings

**Figure 1** *Construction of a normalized cDNA library from porcine sperm RNA.*
   Agarose gel electrophoresis (1.3% agarose) of the N0 and N1 cDNA from porcine sperm RNA (for further details see Example 2).
**Figure 2** *PCR amplification of cDNA inserts of colonies of the normalized cDNA library.*
   About 1,000 colonies of the normalized cDNA library were amplified by PCR in the presence of the 5'-PCR primer used for cDNA amplification and either a primer binding downstream of the *Not* I site (lane 1) or a primer binding upstream of the *Eco* RI site (lane 2).
**Figure 3** *Analysis of cDNA clones of 96 PCR reactions by agarose gel electrophoresis.*
   DNA from wells with amplification products of < 500 bp, without product or with a double band were not sequenced.
**Figure 4** *Comparative characterization of two boar samples.*
   Hybridization images of two arrays, wherein
   Array 1 = semen sample of boar "ADummy",
   Array 2 = semen sample of boar "Bonno".

### Examples

### Example 1: Semen collection and treatment and RNA isolation

### 1. Semen collection

Whole ejaculates and only the sperm-rich fraction were collected from one Duroc boar, using the gloved-hand technique. A total number of 2 ejaculates were collected. At collections the gel portion was removed using double gauge and the ejaculates were subjected to microscopic analyses. Ejaculates were collected on the same day and then immediately placed in a water bath at 37°C. All ejaculates fulfilled the minimum requirements for use in artificial insemination (minimum 70% of motile spermatozoa and 80% of morphologically normal spermatozoa and a total number of spermatozoa higher than 10 ×10⁹).

### 2. Sperm preparation

Sperm-rich fraction of the ejaculate was transferred to 50-ml tubes and centrifuged (2,000 x g, 30 min, room temperature). The resulting pellet was resuspended in PBS and sperm concentration was measured by counting in a Neubauer improved chamber. Sperm concentration was adjusted to 100 million sperms per ml with hypotonic solution (supplemented with 0.5% Triton X-100 to destroy somatic cells):

| 10 mMTris HCl, pH 8.4 | |
|---|---|
| 50 mM | KCl |
| 2,5 mM | MgCl₂ |
| 4 mM | DTT (Dithiotreitol) |
| 0,05% SDS (Sodiumdodecylsulfate) | |

Samples were incubated for 10 min on ice and centrifuged again (2,000 x g, 10 min, RT). The supernatant was discarded and sperms were washed again in PBS and centrifuged (2,000 x g, 10 min, RT). The supernatant was discarded again and sperm concentration was adjusted to finally 100 million sperms per ml and tube. Samples were stored at -80°C until isolation of RNA.

### 3. RNA-isolation of porcine sperm

Total RNA was isolated from 4 tubes (two from each ejaculate) with total 400 million sperms. TRIZOL® reagent (1,000 µl) was added to each tube followed by 15 sec vortex and solution was pulled through an 18-gauge needle to homogenize the samples. Samples were vortexed again for 15 sec and centrifuged (10 min, 12,000 x g, 4°C). The supernatant (950 µl) was transferred to a new 1,5 ml tube and mixed with 200 µl ice cold chloroform, vortexed for 15 sec and incubated for 10 min at room temperature. Following a second centrifugation step (10 min, 12,000 x g, 4°C), the upper aqueous phase (∼600 µl) was transferred to a new tube and gently mixed with 2 µl of glycogen solution, 1 volume 100% isopropanol and incubated for 10 min at room temperature. Samples were centrifuged as described before. The pellet was washed in 1,000 µl 75% ethanol and centrifuged again for 5 min. The pellet was dried on air, resuspended in 17 µl nuclease-free water, heated for 10 min at 55°C and chilled on ice. A DNaseI digestion step was performed using 2 µl DNaseI digestion buffer and 1 µl DNaseI (1 IU/µl) at 25°C for 15 min. DNaseI was inactivated with 1 µl EDTA (25 mM) and denaturation at 65°C for 10 min.

### 4. Storage and transport

On ice on the same day

### Example 2: Construction of a normalized cDNA library from porcine sperm RNA

### 1. Analysis of RNA

Prior to cDNA synthesis the quality of the provided RNA was analyzed by UV spectrophotometry. As the RNA was completely needed for cDNA synthesis, no further quality check was performed.

### 2. cDNA synthesis

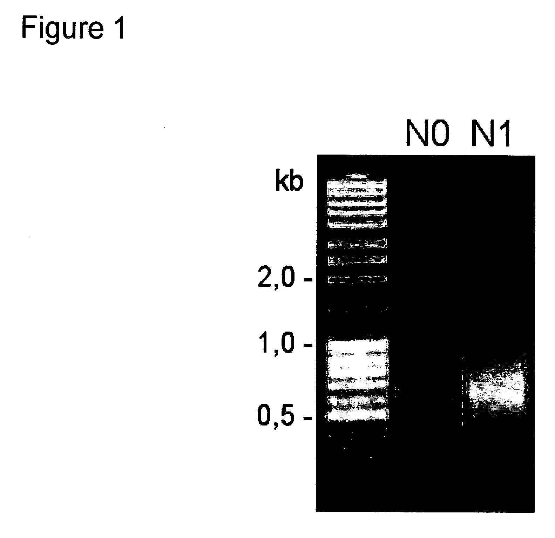

Starting material was poly(A)⁺ RNA purified from the total RNA. An oligo(dT)-Not I primer was used for first strand synthesis. The resulting N0 cDNA was amplified with 26 cycles of LA-PCR (long and accurate PCR; see Figure 1, lane N0).

### 3. Normalization

Normalization was carried out by one cycle of denaturation and reassociation of the cDNA, resulting in N1-cDNA. Reassociated double-stranded cDNA was separated from the remaining single-stranded cDNA (normalized cDNA) by passing the mixture over a hydroxylapatite column. After hydroxylapatite chromatography an additional selection for poly A⁺-containing cDNAs was carried out by oligo(dT) chromatography of the normalized ss-cDNAs prior to amplification with 13 LA-PCR cycles (see Figure 1, lane N1).

### 4. Cloning

For directional cloning, the N1 cDNA was first subjected to a limited exonuclease treatment to generate *EcoRI* overhangs at the 5'-ends and was then digested with *Not* I. Prior to cloning, the N1 cDNA was size-fractionated. For that purpose, the cDNA was separated on a 1.3 % agarose gel. Following elution of cDNAs greater than 0.4 kb, the cDNA was ligated into *EcoRI* and *NotI* digested pBS II sk+ vector. The following adapter sequences remain attached to the 5'- and 3'-ends of the cDNAs.
5'-end (*EcoRI* site): 5'-GAATTCGTGAGCCAGAGGACGAGACAAGTT-3'
   [SEQ ID NO. 1]
3'-end (*NotI* site): 5'-GCGGCCGCTCG(T)₂₅-3'
   [see SEQ ID NO. 2]

The cDNA inserts can be released from the pBS II-vector by a *EcoRI* / *NotI* digestion (underlined bases). Ligations were electroporated into TransforMaxTMEC100TM-T1R (Epicentre) electrocompetent cells. After transformation, glycerol was added to a final concentration of 15 % (v/v) and the cells were frozen at -70 °C in 8 x 550 µl aliquots. After a freeze-thaw cycle, the titer of the library was determined to be about 1.000 cfu per µl bacterial suspension. In total, a number of about 4.400.000 recombinant clones was achieved.

### 5. Quality control - Mass Colony PCR

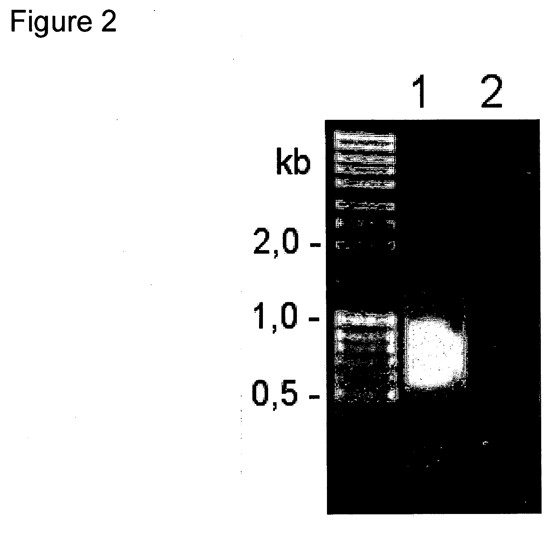

To get a comprehensive impression on the distribution of the insert sizes within the library, about 1,000 colonies grown overnight on a Petri dish were suspended in water. With an aliquot of the bacterial suspension PCR analysis was performed in the presence of the 5'-PCR primer used for cDNA amplification and a primer binding to the T3 promoter of the pBS II sk+ vector 50 bp downstream from the *Not* I site. Therefore, together with the insert 50 bp of vector DNA are co-amplified. The PCR products obtained (Fig.2, lane 1) well reflect the size distribution of the normalized cDNA (Fig.1, lane 2). When the T3-specific primer is replaced with a primer that binds upstream from the *Eco* RI site to the T7 promoter of the vector no amplification of cDNA inserts was observed (Fig. 2, lane 2). This demonstrates that cDNA inserts are in correct directional orientation. In both cases PCR products were analyzed on a 1.3% agarose gel after 23 cycles.

### 6. Picking of cDNA clones

After plating a part of the normalized library on Luria broth (LB) agar plates (12 x 12 cm) containing ampicillin (100 µg/ml), 4,224 bacterial clones were picked and grown overnight at 37 °C in 100 µl LB medium in 96-well microtiter plates. Bacterial suspensions were diluted 20-fold in TE buffer (pH 8.0), incubated at 96 °C for 15 min. After lysis of the bacteria and release of the plasmids the plates were covered with alu foil, frozen on dry ice and stored at -20 °C.

### 7. PCR amplification and sequencing of cDNA inserts

Two microliters of bacterial dilutions were used as template for amplification of the cDNA insertions via PCR in 96-well cycle plates. Reactions were preformed in two steps. In the first step the reaction volume was 20 µl and 5 µl reaction mix were added in a second step to obtain maximal amounts of PCR product. Eighteen microliters of PCR reaction mix were pipetted into each well of 96-well PCR plates. Subsequently, 2 µl of lyzed bacterial suspension was added to each well.

**Table 1. Reaction mix 1**

| **Component** | **For 1 reaction [**µ**l]** | **For 100 reactions [**µ**l]** |
|---|---|---|
| NLT7 15 pmol/µl | 0.5 | 50 |
| LT3 15 pmol/µl | 0.5 | 50 |
| DNTP's 10 mM | 0.5 | 50 |
| 10X reaction buffer | 2.0 | 200 |
| Taq Polymerase (peQLab) 5 U/µl | 0.125 | 12.5 |
| ddH₂O | 14.375 | 1437.5 |
| Total volume | 18 | 180 |

**Table 2. Reaction mix 2**

| **Component** | **For 1 reaction [**µ**l]** | **For 100 reactions [**µ**l]** |
|---|---|---|
| 10X reaction buffer | 0.5 | 50 |
| ddH₂O | 4.375 | 437.5 |
| Taq Polymerase | 0.125 | 12 |
| Total volume | 5 | 500 |

| PCR programm step 1 | | | |
|---|---|---|---|
| Step | Temperature | Time [s] | Link to step # |
| 1 | 96°C | Pause | |
| 2 | 96°C | 120 | |
| 3 | 94°C | 25 | |
| 4 | 64°C | 25 | |
| 5 | 72°C | 90 | 3 (24x) |
| 6 | 8°C | Pause | |

Subsequently, 5 µl PCR reaction mix 2 were added.

| PCR program step 2 | | | |
|---|---|---|---|
| Step | Temperature | Time [s] | Link to step # |
| 1 | 94°C | Pause | |
| 2 | 94°C | 25 | |
| 3 | 64°C | 25 | |
| 4 | 72°C | 90 | 2 (14x) |
| 5 | 20°C | Pause | |

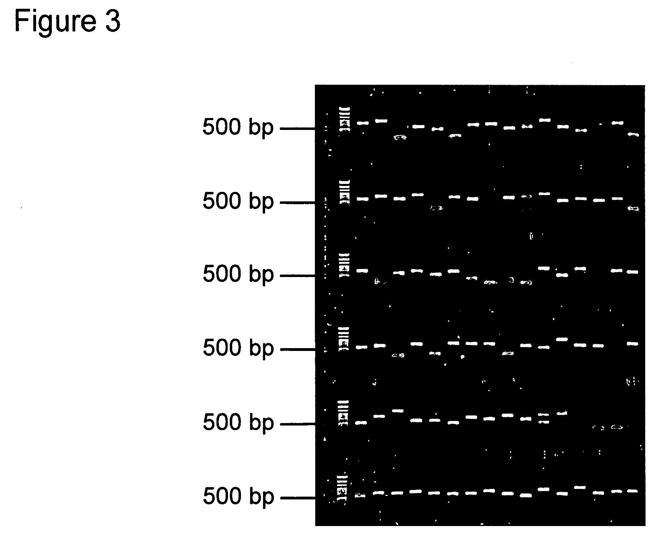

From each PCR reaction, 0.5 µl was analyzed in a TBE agarose gel (0.8%). Per gel 96 PCR reactions were analyzed together with a DNA fragment length standard (100 bp Ladder Plus - Fermentas; 100 ng). A representative gel stained with ethidium bromide is shown in Figure 3.

PCR products smaller than 500 bp, without detectable fragment, or with a double band were not used for sequence analysis. 3,223 PCR reactions were dialyzed to remove PCR primers, dNTPs, and buffer salts. A pipetting robot was used to pipet aliquots of 3 µl from each PCR reaction to a ∅ 47 mm filter (0.25) µm pore size, Millipore). The filter was placed on 40 ml 0.25 x TE (10 ml TE and 30 ml water for 1 h at RT under continuous stirring (200 U/min). The dialyzed PCR reaction was filled with water to a final volume of 10 µl. Sequencing was performed by GATC (Konstanz) with a modified T7 primer, which binds in the plasmid vector upstream of the 5' end of the cDNA fragment.

### 8. Analysis of sequenced clones

The obtained cDNA sequences were compared with public sequence databases using the basic local alignment search tool ("discontiguous Mega BLAST") at the National Center for Biotechnology Information (www.ncbi.nlm.nih.gov/blast/blast/cgi). Complementary DNAs without similar entries in the "nr" database were in addition compared with the "est" database.

### 9. Preparation of cDNA arrays

Fifteen microlitres of PCR products of the cDNA fragment were transferred to 384-well microtiter plates (Abgene, Epsom, Surrey, UK) containing 15 µl 2-fold spotting buffer (40 mN Tris-HCI pH 8.0, 2 M NaCl, 2 mM EDTA, bromphenol blue). 3072 cDNA fragments were distributed on a set of two array spotted on nylon membranes (Nytran plus, Whatman Schleicher & Schuell, Dassel, Germany) on an area of 20 x 50 mm using a microarray roboter (Omnigird Accent, GeneMachines) and solid pins (SSP015, diameter 0.015 inches; Telechem International, Sunnyvale, CA, USA). Spotting was done six times for each PCR product on the same position for sufficient and equal application. Ten nylon membranes were produced simultaneously for each array. The spotted DNA was denatured by incubation with 0.5 N NaOH for 20 min at room temperature. Subsequently, DNA was immobilized by baking for 30 min at 80°C and UV-crosslinking (120 mJ/cm2) (XL-1500 UV Crosslinker; Spectronics Corp., New York, USA).

### Example 3 Hybridization with selected samples

### 1. Semen collection and treatment

Semen samples of two boars were collected and treated as described in Example 1. One of the boars ("ADummy") showed polyspermy in *in vitro* fertilization experiments whereas the other boar ("Bonno") served as normal control.

### 2. RNA isolation

Total RNA was isolated as described in Example 1.

### 3. cDNA synthesis

Double-stranded (ds) cDNA was synthesized using Superscript^{™} III (200 U/µl, Invitrogen) and 50 pmol cDNA synthesis primer (GAGAT20VN; V=A, C or G) for first-strand synthesis. The second strand was sysnthesized with *Escherichia coli* DNA polymerase I (40 U), *E*. *coli* RNase H (2 U) and *E*. *coli* DNA ligase (10 U) (Invitrogen) according to the manufacturer's instructions. Residual RNA was digested with 5 µl RNase (0.5 mg/µl, DNase-free, from bovine pancreas; Roche Diagnostics) for 90 min at 37 °C. Remaining nucleotides and primers were removed using Microspin S200-HR spin columns (GE Healthcare Life Science, Munich, Germany) and after that cDNA was precipitated with 15 µl 3 M NaOAc and 150 µl Isopropanol. Dry sediment was solubilized in 20 µl 0.5 x TE (10 mM Tris/HCI, pH 8.0; 0.1 mM EDTA) buffer (pH 8.0). Three replicates for each boar were made. The quality of cDNA was determined by agarose gel electrophoresis.

### 4. Probe generation

³³P-labeled cDNA probes were generated from ds cDNA. Complementary DNA was heat-denatured for 10 min at 96°C and then chilled on ice. High Prime reaction mixture (Roche), dNTP mixture (cCTP final concentration 10 pmol/µl, dATP, dGTP and dTTP final concentration each 100 pmol/µl) and 90 µCi [α-33P]dCTP were added to a final volume of 20 µl. Reactions were incubated for 1 h at 37 °C, heat-inactivated for 20 min at 65 °C and purified with ProbeQuant G50 spin columns (GE Healthcare Life Sciences) to remove unincorporated nucleotides ant to estimate labeling efficiency.

### 5. Hybridization

Hybridization was done as follows: pre-hybridization was done for six arrays together in one 15 cm glass hybridization bottle, respectively: 2 x10 min 10 ml 0.1 x PBS / 1% SDS at 85 °C; 3 x 10 min 10 ml 1 x PBS / 10 % SDS at 65 %; 1 x 10 min 10 ml 1 x PBS / 10 % SDS at room temperature. Hybridization probes were denatured for 15 min at 96 °C immediately before adding to the hybridization solution. Hybridization was done in plastic vials (Poly-Q vials, 18 ml; Beckman Coulter, Munich; Germany) in 2.5 ml 1 x PBS, pH 7.5 / 10 % SDS for 47 h at 65 °C. Arrays 1 and 2 were hybridized in the same vial.

After hybridization, the arrays were put together in 15 cm glass hybridization bottle, six arrays per bottle, and washed as follows: 3 x 5 min 10 ml 1 x PBS / 10 % SDS at 65 °C; 3 x 10 min 10 ml 1 x PBS / 10 % SDS at 65 °C; 3 x 5 min 10 ml 1 x PBS / 1 % SDS / 2 mM EDTA at 30 °C. Filters were dried by baking at 80 °C for 20 min.

The Filters were exposed to an imaging plate BAS-SR (Fuji Photo Film Co.). Imaging plates were scanned with a phosphor imager (Typhoon Imager, GE Healthcare Life Sciences). Labeling and hybridization was done in parallel for all six sample.

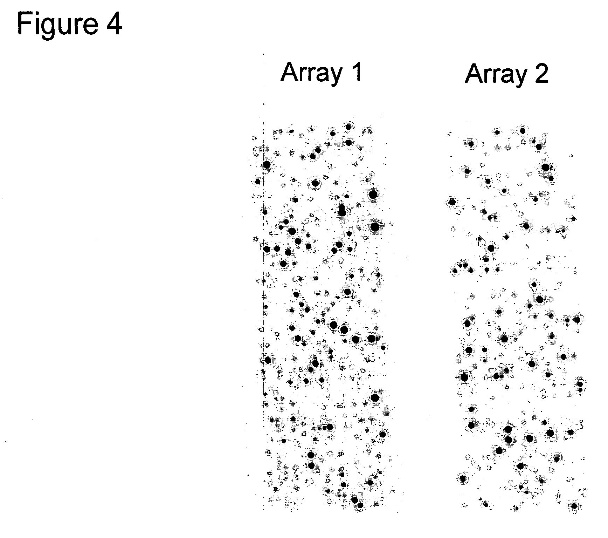

See figure 4 for Array 1 and Array 2.

### 6. Analysis of array data

Array evaluation was done using AIDA Image Analyzer (Version 4.15, Raytest, Straubenhardt, Germany), background was subtracted with the 'Lowest Grid Dot' function. Raw data were normalized with the BioConductor package vsn. After log2-transformation a significance analysis was performed using the 'significance analysis of microarrays method' (SAM).

### 7. Results

Table 3 shows part of the list of genes which are expressed higher in boar "ADummy" (Array 1) compared to boar "Bonno" (Array 2).

**Table 3. Differential expressed genes**

| Databank Hit | Gene Symbol | Access Number |
|---|---|---|
| Sus scrofa mitochondrion, complete genome Length=15978 | 12S rRNA | AF486866 |
| Sus scrofa mitochondrion, complete genome Length=16613 | ATPase 6 | AF034253 |
| Sus scrofa mitochondrion, complete genome Length=16613 | ATPase 8 | AF034253 |
| Sus scrofa mitochondrion, complete genome Length=16613 | COI | AF034253 |
| Sus scrofa mitochondrion, complete genome Length=16613 | COII | AF034253 |
| Sus scrofa mitochondrion, complete genome Length=16613 | COIII | AF034253 |
| Sus scrofa isolate PN149 cytochrome b gene, complete cds, mitochondrial Length=1140 | CYTB | EF061504 |
| Sus scrofa mitochondrion, complete genome Length=16613 | NADH1 | AF034253 |
| Sus scrofa mitochondrion, complete genome Length=16613 | NADH2 | AF034253 |
| SSJ002189 Sus scrofa complete mitochondrial DNA Length=16680 | NADH4 | AJ002189 |
| Sus scrofa mitochondrion, complete genome Length=16613 | NADH5 | AF034253 |
| Sus scrofa mitochondrion, complete genome Length=16613 | NADH6 | AF034253 |
| Sus scrofa mRNA, clone:AMP010048E04, expressed in alveolar macrophage Length=710 | | AK230819 |

### References

Aasen E and Medrano JF (1990). Amplification of the ZFY and ZFX genes for sex identification in human, cattle, sheep and goats. Bio/Technology. 8: 1279-1281.
Amanai M, Brahmajosyula M, Perry AC (2006) A restricted role for sperm-borne microRNAs in mammalian fertilization. Biol Reprod 75: 877-884. Epub 2006 Aug 30.
Balhorn R, Brewer L, Corzett M and Cosman J (1999) Protamine-mediated condensation of DNA and the subunit structure of sperm chromatin. Biol Reprod 60: M34.
Berezikov E, Cuppen E, Plasterk RH (2006) Approaches to microRNA discovery. Nat Genet 38 Suppl: S2-7.
Esquela-Kerscher A, Slack FJ (2006) Oncomirs - microRNAs with a role in cancer. Nat Rev Cancer 6(4): 259-269.
Gledhill BL (1988). Selection and separation of X- and Y- chromosome-bearing mammalian sperm. Gamete Res. 20(3): 377-395.
Grivna ST, Pyhtila B and Lin H (2006) MIWI associates with translational machinery and PIWI-interacting RNAs (piRNAs) in regulating spermatogenesis. Proc Natl Acad Sci U S A 103(36): 13415-13420.
Johnson LA, Flook JP and Hawk HW (1989). Sex preselection in rabbits: live births from X and Y sperm separated by DNA and cell sorting. Biol Reprod. 41(2): 199-203.
Kim VN, Nam JW (2006) Genomics of microRNA. Trends Genet 22(3): 165-173.
Kotaja N, Bhattacharyya SN, Jaskiewicz L, Kimmins S, Parvinen M, Filipowicz W and Sassone-Corsi P (2006) The chromatoid body of male germ cells: similarity with processing bodies and presence of Dicer and microRNA pathway components. Proc Natl Acad Sci U S A 103(8):2647-2652.
Krawetz SA (2005) Paternal contribution: new insights and future challenges. Nat Rev Genet 6: 633-642.
Krutzfeldt J, Poy MN, Stoffel M (2006) Strategies to determine the biological function of microRNAs. Nat Genet 38 Suppl: S14-19.
Miller D, Briggs D, Snowden H, Hamlington J, Rollinson S, Lilford R and Krawetz SA (1999) A complex population of RNAs exists in human ejaculate spermatozoa: implications for understanding molecular aspects of spermiogenesis. Gene 237: 385-392.
Miller D, Ostermeier GC and Krawetz SA (2005) The controversy, potential and roles of spermatozoal RNA. Trends Mol Med 11: 156-163.
Miller D and Ostermeier GC (2006) Towards a better understanding of RNA carriage by ejaculate spermatozoa. Hum Reprod 12(6): 757-767.
Ostermeier GC, Dix DJ, Miller D, Khatri P and Krawetz SA (2002) Spermatozoal RNA profiles of normal fertile men. Lancet 360: 772-777.
Ostermeier GC, Goodrich RJ, Moldenhauer JS, Diamond MP and Krawetz SA (2005) A suite of novel human spermatozoal RNAs. J Androl 26: 70-74.
Rajewsky N (2006) microRNA target predictions in animals. Nat Genet 38 Suppl: S8-13.
Seidel GE (1999). Sexing mammalian sperm and embryos - state of the art. J Reprod Fertil. 54, 475-485.
Steger K (2001) Haploid spermatids exhibit translationally repressed mRNAs. Anat Embryol 203: 323-334.
Wang H, Zhou Z, Xu M, Li J, Xiao J, Xu ZY and Sha J (2004) A spermatogenesis related gene expression profile in human spermatozoa and its potential clinical applications. J Mol Med 82: 317-324.
Zhao Y, Li Q, Yao C, Wang Z, Zhou Y, Wang Y, Liu L, Wang Y, Wang L and Qiao Z (2006) Characterization and quantification of mRNA transcripts in ejaculated spermatozoa of fertile men by serial analysis of gene expression. Hum Reprod 21: 1583-1590.

## Claims

1. Method for categorizing a sample containing spermatozoa comprising determining a RNA expression profile in said sample by hybridization and/or sequencing techniques, wherein the RNA is selected from messenger RNA (mRNA), noncoding RNA (ncRNA) and micro RNA (miRNA).

2. Method according to claim 1, wherein the sample containing spermatozoa is ejaculate, semen, sperm-rich fraction of ejaculate, sperm cells from the epidydimis, sperm cells or their progenitors from the testis.

3. Method according to claim 1 or claim 2, wherein the donor of the sample containing spermatozoa is an animal, a mammal, a bird, livestock or a breeding animal.

4. Method according to claim 3, wherein the donor of the sample containing spermatozoa is a boar, a bull, a stallion, a rooster, a male dog or a male.

5. Method according to any of the preceding claims, wherein the hybridization is carried out by using nucleic acid arrays, preferably cDNA arrays or oligonucleotide arrays.

6. Method according to any of the preceding claims, wherein determining a RNA expression profile comprises determining one or more of the following:
- presence, frequency and/or concentration of RNA,
- differences in RNA sequence,
such as deletions, polymorphisms and SNP genotyping,
- differences in RNA length,
- alternative usage of exons and introns.

7. Method according to any of the preceding claims, wherein the sequencing techniques are selected from sequencing of normalized cDNA libraries and sequencing of the whole profile by high-throughput sequencing technologies, such as 454 technology.

8. Method according to any of the preceding claims, wherein determining a RNA expression profile furthermore comprises quantitative analysis, such as quantitative analysis of candidate RNAs.

9. Method according to any of the preceding claims, comprising the following steps:
a) providing a sample containing spermatozoa,
b) isolating total RNA from said sample,
c) synthesizing cDNA from the RNA isolated in step b),
d) generating labelled probes from the cDNA obtained in step c),
e) providing a nucleic acid array,
f) hybridizing the probes generated in step d) with the array of step e),
g) obtaining a RNA expression profile of the sample from the hybridization signals obtained in step f),
h) comparing the RNA expression profile of the sample with the RNA expression profile of a control sample,
i) categorizing the sample according to the RNA expression profile obtained in step g) and/or from the results of the comparison of step h),
j) optionally, obtaining subpopulations of the sample.

10. Method according to claim 9, wherein the probes in step d) are cDNA or cRNA, preferably single stranded cDNA (ss cDNA), double stranded cDNA (ds cDNA) or single stranded cRNA (ss cRNA).

11. Method according to claim 9 or claim 10, wherein the labelled probes in step d) are generated by
- labelling with radioactive or non-radioactive labels,
- incorporating of dNTPs with reactive side groups, and/or
- incorporating of characteristic nucleotide sequence(s).

12. Method according to claim 11, wherein the radioactive label is a radio-isotope which is selected from ³³P or ³²P.

13. Method according to claim 11, wherein the non-radioactive label is as fluorescent dye or a luminescent dye.

14. Method according to claim 11, wherein the non-radioactive label is selected from biotin, digoxigenin and avidin.

15. Method according to claim 11 or claim 12, wherein the labelled probes are generated by random primed labelling.

16. Method according to any of claims 9 to 15, wherein the nucleic acid array provided in step e) is a cDNA array obtained from a normalized spermatozoa cDNA library.

17. Method according to claim 16, wherein the cDNA array obtained from a normalized spermatozoa cDNA library was made by
- providing a sample containing spermatozoa,
- isolating total RNA from said sample,
- synthesizing cDNA from the RNA isolated,
- normalization of the cDNA obtained,
- cloning of the normalized cDNA into a plasmid vector,
- propagation of individual clones,
- determining the nucleotide sequence of cloned cDNA fragments,
- selection of a representative clone collection by discarding multiple occurring clones,
- preparation of cloned cDNA fragments,
- spotting of cDNAs onto a carrier material,
- denaturing ds cDNA and fixation of ss cDNA on the carrier material.

18. Method according to claim 16 or claim 17, wherein the cDNA array obtained from a normalized spermatozoa cDNA library was made from another sample containing spermatozoa which was obtained from the same animal species as the sample provided in step a).

19. Method according to any of claims 9 to 18, wherein in step i) the sample is categorized with respect to fertility, subfertility, infertility, fecundity, breeding selection, polyspermy, and subpopulations of the sample donor.

20. Method according to claim 19, wherein the subpopulations of the sample differ in the type of sex chromosome of the spermatozoa.

21. Method according to any of the preceding claims, further comprising the steps
- obtaining a translation product profile of the sample and
- categorizing the sample according to the translation product profile,
wherein these steps are performed after step g), h) and/or i).

22. Method according to any of the preceding claims, further comprising the step
- distinguishing between male (Y-bearing) and female (X-bearing) spermatozoa of said sample.

23. Method according to claim 22, further comprising the step
- separating male (Y-bearing) from female (X-bearing) spermatozoa.

24. Male (Y-bearing) spermatozoon or spermatozoa obtained by a method according to claim 23.

25. Female (X-bearing) spermatozoon or spermatozoa obtained by a method according to claim 23.

26. Categorized sample obtained by a method according to any of claims 1 to 21.

27. Categorized subpopulation of a sample obtained by a method according to any of claims 1 to 22.

28. Categorized spermatozoon or spermatozoa obtained by a method according to any of claims 1 to 23.

29. Use of a RNA expression profile of a sample containing spermatozoa as selection criterion for fertility, subfertility, infertility, fecundity, breeding selection, and breeding selection based on fertility or for determining the sex chromosome of spermatozoa.

30. Use of a RNA expression profile of a sample containing spermatozoa for obtaining a translation product profile of the sample.

31. Use according to claim 30, wherein the RNA expression profile was obtained by a method according to any of claims 1 to 20.

32. Use of a translation product profile of a sample containing spermatozoa as selection criterion for fertility, subfertility, infertility, fecundity, breeding selection, and breeding selection based on fertility or for determining the sex chromosome of spermatozoa.

33. Use according to claim 32, wherein the translation product profile was obtained by a method according to any of claims 1 to 21.

34. Use according to any of claims 29 to 33, wherein the sample containing spermatozoa is ejaculate, semen, sperm-rich fraction of ejaculate, sperm cells from the epidydimis, sperm cells or their progenitors from the testis.

35. Use according to any of claims 29 to 34, wherein the donor of the sample containing spermatozoa is an animal, a mammal, a bird, livestock or a breeding animal.

36. Use according to any of claims 29 to 35, wherein the donor of the sample containing spermatozoa is a boar, a bull, a stallion, a rooster, a male dog or a male.


SEQUENCE LISTING

<110>  Minitüb Abfüll- und Labortechnik GmbH & Co KG

<120>  Method for categorizing samples containing spermatozoa by molecular
       profiling

<130>  FB19165(R)

<160>  2

<170>  PatentIn version 3.2

<210>  1
<211>  30
<212>  DNA
<213>  Artificial

<220>
<223>  5' adapter sequence

<400>  1
gaattcgtga gccagaggac gagacaagtt                                        30


<210>  2
<211>  36
<212>  DNA
<213>  Artificial

<220>
<223>  3' adapter sequence

<400>  2
gcggccgctc gttttttttt tttttttttt tttttt                                 36